# EUROPEAN PATENT APPLICATION

(11) **EP 4 410 207 A1**
(43) Date of publication of application: **07.08.2024**
(21) Application number: 22876146.6
(22) Date of filing: 26.09.2022
(51) Int. Cl.: A61B 5/257

(54) **BIOSENSOR**

(30) Priority: 28.09.2021 JP 2021157511; 28.03.2022 JP 2022051477
(71) Applicant: Nitto Denko Corporation, Ibaraki-shi, Osaka 567-8680 (JP)
(72) Inventor: NISHIYAMA, Keine, Ibaraki-shi, Osaka 567-8680 (JP)
(74) Representative: Hoffmann Eitle
(86) International application number: PCT/JP2022/035772
(87) International publication number: WO 2023/054279

(57) **Abstract**

A biological sensor includes a sensor body configured to obtain biological information; a cover member having a housing space and an opening of the housing space, the sensor body being housed in the housing space; a first sheet member provided to face the opening in the cover member; and a second sheet member provided to face the cover member with the first sheet member being between the second sheet and the cover member. At least a part of an outer periphery of the first sheet member projects with respect to outer peripheries of the cover member and the second sheet member. Thereby, it is possible to provide a biological sensor that can be suppressed from being detached from a living body, and can suppress reduction in attachment sensation due to deformation of skin caused by a body movement of the living body.

## Description

### TECHNICAL FIELD

The present invention relates to biological sensors.

### BACKGROUND ART

Wearable biological sensors configured to be attached to a living body and obtain biological information, such as electrocardiographic signals and the like, are known. For example, a sensor sheet for attaching this type of biological sensor to the living body includes an upper sheet, a lower sheet larger than the upper sheet, and an electrical circuit (substrate and interconnects) disposed between the upper sheet and the lower sheet. The lower sheet is formed to be thinner than the upper sheet in order to ensure stretchability for conforming to stretch of skin. The upper sheet is attached to the lower sheet with the electrical circuit being held therebetween. The sensor sheet is attached to the living body via a tacky layer provided at a surface of the lower sheet that is opposite to the electrical circuit (see, for example, PTL 1).

### CITATION LIST

### PATENT LITERATURE

PTL 1: Japanese Patent No. 6537618

### SUMMARY OF INVENTION

### TECHNICAL PROBLEM

When the upper sheet is attached to the lower sheet formed to be thin to have increased stretchability, the upper sheet having a strength higher than in the lower sheet also stretches in conformity to the lower sheet that is stretching along with stretch of skin. As a result, the stretch of skin is restricted by the upper sheet. Also, when an electronic circuit having a sensor function is disposed between the upper sheet and the lower sheet in addition to the substrate and interconnects, the upper sheet and the lower sheet need to have predetermined hardness in order to protect the electronic circuit from an external force and suppress reduction in measurement accuracy. When the lower sheet having low elasticity is attached to skin, the stretch of skin is restricted by the lower sheet. In this way, when the stretch of skin is restricted by the sheet, unpleasant feelings arise upon the stretch of skin, and attachment sensation of the biological sensor is degraded.

The present invention has been accomplished in view of the above, and it is an object of the present invention to provide a biological sensor that can be suppressed from being detached from a living body and suppress degradation in attachment sensation with respect to deformation of skin caused by a body movement of the living body.

### SOLUTION TO PROBLEM

A biological sensor of an embodiment of the present invention includes a sensor body configured to obtain biological information; a cover member having a housing space and an opening of the housing space, the sensor body being housed in the housing space; a first sheet member provided to face the opening in the cover member; and a second sheet member provided to face the cover member with the first sheet member being between the second sheet and the cover member. At least a part of an outer periphery of the first sheet member projects with respect to outer peripheries of the cover member and the second sheet member.

### ADVANTAGEOUS EFFECTS OF INVENTION

According to the disclosed technique, it is possible to provide a biological sensor that can be suppressed from being detached from a living body and suppress degradation in attachment sensation with respect to deformation of skin caused by a body movement of the living body.

### BRIEF DESCRIPTION OF DRAWINGS

[FIG. 1] FIG. 1 is an entire configuration diagram illustrating an example of a biological sensor according to a first embodiment.
[FIG. 2] FIG. 2 is a plan diagram illustrating examples of parts of the biological sensor of FIG. 1.
[FIG. 3] FIG. 3 is an exploded cross-sectional diagram illustrating a longitudinal cross section of the biological sensor of FIG. 1.
[FIG. 4] FIG. 4 is a schematic diagram illustrating the longitudinal cross section of the biological sensor of FIG. 1.
[FIG. 5] FIG. 5 is an explanatory diagram illustrating a state in which the biological sensor of FIG. 1 is attached to the chest of a living body.
[FIG. 6] FIG. 6 is a table describing examples of evaluation results of detachment endurance of the biological sensor against stretch of skin.
[FIG. 7] FIG. 7 is a plan diagram illustrating examples of shapes of a cover, an upper sheet, and a lower sheet of a biological sensor according to a second embodiment.

### DESCRIPTION OF EMBODIMENTS

In the following, embodiments of the invention will be described with reference to the drawings. In the drawings, the same components are denoted by the same reference numerals and duplicate description thereof may be omitted.

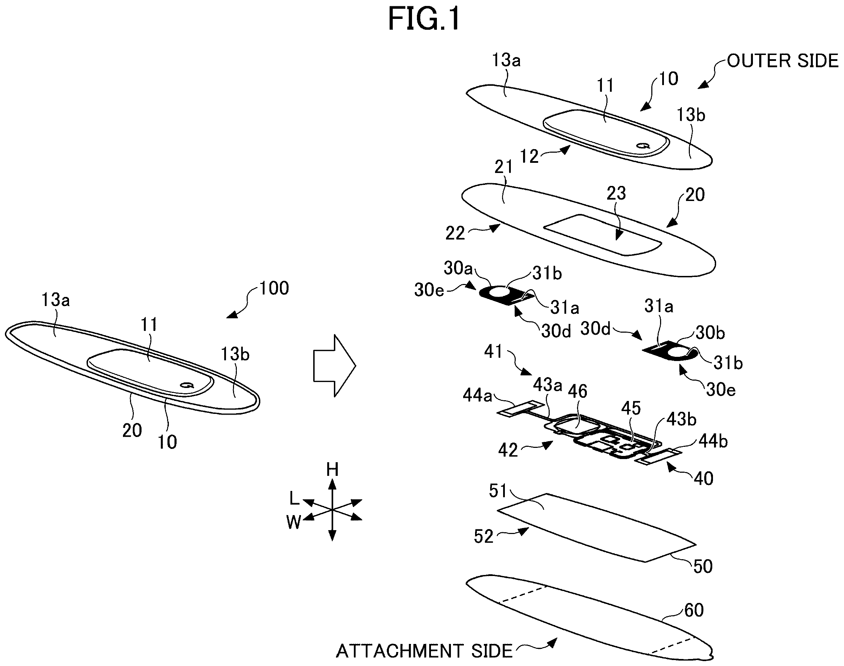

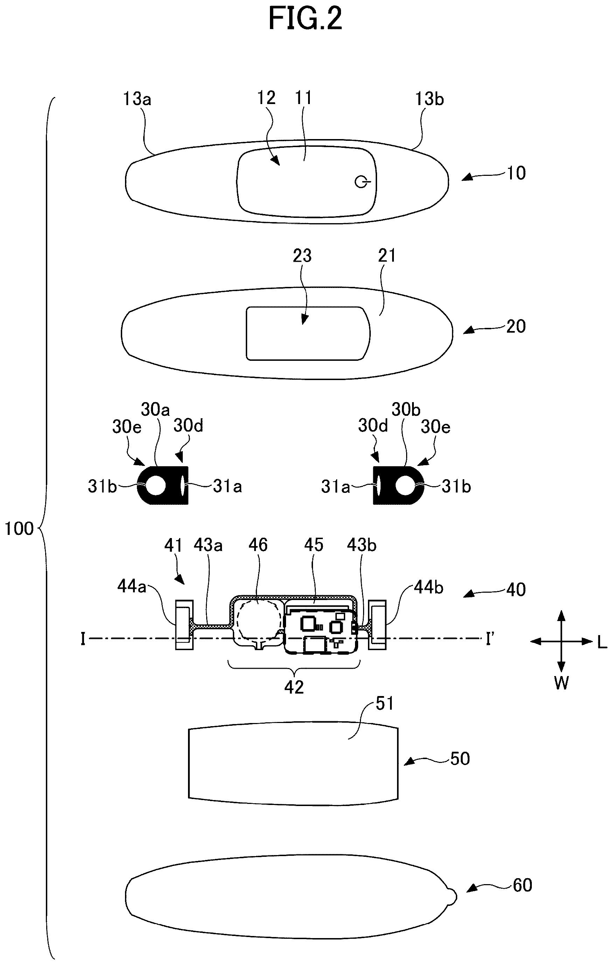

FIG. 1 is an entire configuration diagram illustrating an example of the biological sensor according to the first embodiment. A left-hand part of FIG. 1 illustrates an outer appearance of a biological sensor 100, and a right-hand part of FIG. 1 illustrates a state in which the parts of the biological sensor 100 are exploded in the order of stacking. FIG. 2 is a plan diagram illustrating examples of the parts of the biological sensor 100 of FIG. 1.

The biological sensor 100 as illustrated in FIG. 1 and FIG. 2 has a thin and long shape, and is formed by stacking a cover 10, an upper sheet 20, electrodes 30a and 30b, a sensor portion 40, a lower sheet 50, and a release sheet 60. As understood from FIG. 1 and FIG. 2, the cover 10, the upper sheet 20, and the release sheet 60 have a thin and long shape.

In the following, when the electrodes 30a and 30b are not distinguished for description, the electrodes 30a and 30b will also be referred to as an electrode 30. Further, in the following, a surface of the biological sensor 100 that is to be attached to a living body (subject) (the release sheet 60 side) will be referred to as an attachment side, and a surface thereof opposite to the attachment side (the cover 10 side) will be referred to as an outer side. The cover 10 is an example of the cover member, the upper sheet 20 is an example of the first sheet member, and the lower sheet 50 is an example of the second sheet member.

The sensor portion 40 includes a flexible substrate 41 (resin substrate) in which various parts configured to obtain biological information are mounted. In the flexible substrate 41, a sensor body 42, narrow portions 43a and 43b, and terminals 44a and 44b are integrally formed. The terminals 44a and 44b are respectively connected to the sensor body 42 via the narrow portions 43a and 43b. In the following, when the narrow portions 43a and 43b are not distinguished for description, the narrow portions 43a and 43b will also be referred to as a narrow portion 43. Also, when the terminals 44a and 44b are not distinguished for description, the terminals 44a and 44b will also be referred to as a terminal 44.

The sensor body 42 includes a parts-mounting portion 45, and a battery-receiving portion 46 that is configured to receive a battery of a coin type or the like. The battery for use is, for example, CR2025.

The cover 10 is formed of, for example, a material having flexibility, such as a silicone resin (hardness: shore A40) or the like. The cover 10 may be formed of a fluororesin (fluorine rubber), a urethane resin (urethane rubber), styrene-butadiene rubber (SBR), or the like. The cover 10 includes a projection 11 at a center portion in a longitudinal direction L, and the projection 11 projects toward the outer side in a height direction H. The inner side (attachment side) of the projection 11 forms a housing space 12 that houses the sensor body 42 and the opening. The attachment side of the cover 10 has a flat shape.

The thicknesses of an upper surface and a side wall of the projection 11 are larger than the thicknesses of flat portions 13a and 13b that are provided at both ends of the cover 10 in the longitudinal direction L. Thereby, flexibility (stretchability) of the projection 11 can be made lower than flexibility of the flat portions 13a and 13b, and it is possible to protect the parts mounted in the sensor body 42 from the external force applied to the biological sensor 100. No particular limitation is imposed on the thicknesses of the upper surface and the side wall of the projection 11. However, for example, the thicknesses of the upper surface and the side wall of the projection 11 are set in a range of from 1.5 through 3 mm (millimeters), and the thicknesses of the flat portions 13a and 13b are set in a range of from 0.5 through 1 mm (preferably 0.8 mm).

Meanwhile, the flat portions 13a and 13b having a small thickness have high flexibility compared to the projection 11. Therefore, when the biological sensor 100 is attached to the skin of the living body (subject), the flat portions 13a and 13b can deform in conformity to deformation of a body surface caused by a body movement (stretching, bending, or twisting). This can reduce stress applied to the flat portions 13a and 13b upon deformation of the body surface, and successfully suppress detachment of the biological sensor 100 from the skin. In the following, when the flat portions 13a and 13b are not distinguished for description, the flat portions 13a and 13b will also be referred to as a flat portion 13.

The outer peripheries of the flat portions 13a and 13b have a shape that gradually decreases in thickness toward the end. This can further increase flexibility of the outer peripheries of the flat portions 13a and 13b, and improve attachment sensation felt when the biological sensor 100 is attached to the living body compared to when the thicknesses of the outer peripheries of the flat portions 13a and 13b are not made small. As described below, the upper sheet 20 has a function of reducing the stress applied to the flat portions 13a and 13b upon deformation of the body surface.

The upper sheet 20 is formed to have a shape that is slightly larger than the cover 10 in a plan view. That is, the outer periphery of the upper sheet 20 projects with respect to the outer periphery of the cover 10 in a state in which the cover 10 is attached. For example, an amount of projection (projected length) of the outer periphery of the upper sheet 20 from the outer periphery of the cover 10 is several millimeters (preferably about 5 mm).

The upper sheet 20 is formed using, for example, a polyolefin-based foamed sheet having an open-cell structure, or a polyurethane sheet or a non-woven fabric sheet. The upper sheet 20 has moisture permeability. The moisture permeability of the upper sheet 20 is higher than the moisture permeability of the cover 10. Both surfaces of the upper sheet 20 are respectively provided with tacky layers 21 and 22. The tacky layers 21 and 22 may be provided by attaching pieces of double-sided tacky tape to the upper sheet 20, or may be provided by coating or spraying a tacky agent to the upper sheet 20. Because of the high moisture permeability of the upper sheet 20, it is possible to efficiently release vapor derived from sweat and the like from the outer periphery of the upper sheet 20 that is not covered by the cover 10. As a result, it is possible to suppress accumulation of sweat between the upper sheet 20 and the skin. This can suppress skin irritation, and suppress detachment of the outer periphery of the upper sheet 20. Note that in order to prevent attachment to clothes and the like, it is preferable not to provide the tacky layer 21 in the outer periphery of the upper sheet 20 that projects with respect to the outer periphery of the cover 10.

For example, the tacky agent used for a tacky layer 22 provided on the attachment side of the upper sheet 20 has moisture permeability. As described below, this can transfer water vapor derived from sweat and the like, generated from the living body to which the biological sensor 100 is attached, to the upper sheet 20 via the tacky layer 22, and release the water vapor to the exterior of the biological sensor 100 from the upper sheet 20.

Here, the thickness of the tacky layer 22 may be changed from position to position on the upper sheet 20. For example, the tacky layer 22 may be formed by repeatedly arranging band portions smaller in thickness than other portions (or band portions having zero thicknesses). Alternatively, the tacky layer 22 may be formed by dotting the tacky agent, or arranging tacky agent-free portions in a dotted manner. The band portion may be formed in a straight-line shape, a wavy shape, or a circular shape. The moisture permeability of the tacky layer 22 increases as the tacky agent becomes thinner. Therefore, by forming the tacky layer 22 in which the tacky agent is partially thin, it is possible to increase moisture permeability while maintaining a tacky force.

No particular limitation is imposed on the thickness of the upper sheet 20 when the foamed sheet or the non-woven fabric sheet is used for the upper sheet 20. The thickness of the upper sheet 20 is, for example, from about 0.5 through about 1.5 mm (preferably 1 mm). As the foamed sheet, for example, a foamed sheet such as FOLEC available from INOAC CORPORATION or the like, is used. As the non-woven fabric sheet, for example, base fabric EW for medical patch available from JAPAN VILENE COMPANY, LTD. is used. When the polyurethane sheet is used for the upper sheet 20, the thickness of the upper sheet 20 is, for example, from about 10 through about 300 um (preferably 30 um). As the polyurethane sheet, for example, ESMER URS available from Nihon Matai Co., Ltd. is used. The material of the tacky layer 21 is, for example, a material having biocompatibility. Examples of such a material include acrylic tacky agents, silicone-based tacky agents, and the like, with silicone-based tacky agents being preferable.

The thickness of the tacky layer 21 is, for example, 10 um or larger and preferably 20 um or larger and is, for example, 95 um or smaller and preferably 70 um or smaller. The material of the tacky layer 22 is, for example, a material having biocompatibility, such as an acrylic tacky agent, a silicone-based tacky agent, or the like, with an acrylic tacky agent being preferable. Examples of the acrylic tacky agent include, for example, acrylic polymers described in Japanese Patent Publication No. 2002-65841. The thickness of the tacky layer 22 is, for example, 10 um or larger and preferably 20 um or larger and is, for example, 95 um or smaller and preferably 60 um or smaller. The stretchability of the upper sheet 20 is set to be higher than the stretchability of the cover 10.

The upper sheet 20 has a through-hole 23 at a position facing the sensor body 42. Owing to the through-hole 23, the sensor body 42 can be housed in the housing space 12 from the opening of the cover 10 without obstruction by the upper sheet 20.

The electrode 30 is, for example, a dry electrode that does not need coating of a conductive gel upon measurement of biological signals. The electrode 30 is formed by coating a conductive polymer onto a resin sheet having a thickness of about several tens of micrometers (e.g., from 20 through 25 um). As the resin sheet, for example, a polyethylene terephthalate sheet having a thickness of 25 um is used. As the conductive polymer, for example, PEDOT-PSS is used. The electrode 30 for use may be a monolayered film of a conductive polymer, including no resin sheet. The electrode 30 is attached to the upper sheet 20 with the tacky layer 22 of the upper sheet 20.

The electrodes 30a and 30b each have: a through-hole 31a provided on one end side (inner side) in the longitudinal direction L and having an oval shape that is thin and long in a widthwise direction (lateral direction) W; and a through-hole 31b provided on the other end side (outer side) in the longitudinal direction L and having a circular shape. The electrode 30a is disposed at a position at which the through-hole 31a faces a terminal 44a and the through-hole 31b is not shielded by the lower sheet 50. The electrode 30b is disposed at a position at which the through-hole 31a faces a terminal 44b and the through-hole 31b is not shielded by the lower sheet 50. The portion enclosing the through-hole 31a of the electrode 30a contacts the terminal 44a, and the portion enclosing the through-hole 31a of the electrode 30b contacts the terminal 44b. In the following, one end side of the electrode 30a to contact the terminal 44a and one end side of the electrode 30b to contact the terminal 44b will be referred to as a facing portion 30d. Also, a through-hole 31b-including portion of the electrode 30a not to contact the terminal 44a and a through-hole 31b-including portion of the electrode 30b not to contact the terminal 44b (the other end sides (outer sides) in the longitudinal direction L) will be referred to as an exposed portion 30e. The electrode 30 enables the tacky layer 22 to be exposed to the attachment side from the through-holes 31a and 31b in a state of being attached to the tacky layer 22.

The lower sheet 50 is formed using, for example, a resin sheet having a thickness of about several tens of micrometers through about 100 um. Both surfaces of the lower sheet 50 are provided with tacky layers 51 and 52. For example, the tacky force of the tacky layers 21 and 22 is higher than the tacky force of the tacky layers 51 and 52. A resin sheet used for the lower sheet 50 has waterproofness that does not permeate moisture and water vapor. That is, the moisture permeability of the upper sheet 20 is higher than the moisture permeability of the lower sheet 50. For example, the lower sheet 50 may be formed such that an outer profile thereof at both sides in the widthwise direction W conforms to an outer profile of the upper sheet 20 at both sides in the widthwise direction W. The lower sheet 50 is formed using, for example, an ethylene-vinyl acetate (EVA) film (thickness: 80 um). For example, an acrylic tacky agent for skin (thickness: 40 um) available from Nitto Denko Corporation is used for the tacky layers 51 and 52.

The lower sheet 50 is formed such that the length thereof in the longitudinal direction L is less than the corresponding length of the upper sheet 20. Both ends of the lower sheet 50 in the longitudinal direction L are formed at positions that hold the terminals 44a and 44b between the lower sheet 50 and the upper sheet 20, and that expose the exposed portion 30e of the electrode 30. That is, the lower sheet 50 is attached to the upper sheet 20 such that the electrode 30 can be partially exposed from both ends of the lower sheet 50 in the longitudinal direction L.

The lower sheet 50 and the portions of the upper sheet 20 exposed from both ends of the lower sheet 50 in the longitudinal direction L form an attachment surface to a living body P. The attachment surface corresponding to the lower sheet 50 is provided with the tacky layer 52, and the attachment surface corresponding to the upper sheet 20 is provided with the tacky layer 22. This can achieve waterproofness/moisture permeability and tackiness differing in accordance with positions on the attachment surface.

The width of the lower sheet 50 (the length thereof in the widthwise direction W) may be smaller than the width of an overlapping portion of the upper sheet 20 facing the lower sheet 50. By making the width of the lower sheet 50 smaller than the width of the upper sheet 20, the tacky layer 22 provided on the upper sheet 20 and having relatively high tackiness can be exposed from around the tacky layer 52 provided on the lower sheet 50 and having relatively low tackiness. This can increase adhesiveness of the biological sensor 100 to the living body P.

Both ends of the tacky layer 51 of the lower sheet 50 in the longitudinal direction L are provided at positions that face the facing portion 30d of the electrode 30. This can hold the terminal 44 and the facing portion 30d of the electrode 30 between the upper sheet 20 and the lower sheet 50 in a compressed state, and achieve an electrical conduction between the electrode 30 and the terminal 44.

The release sheet 60 protects the tacky layers 22 and 52, and the exposed portion 30e of the electrode 30. To do this, the release sheet 60 is attached to the tacky layers 22 and 52 exposed to the attachment side until the biological sensor 100 is attached to the living body.

According to the biological sensor 100 as illustrated in FIG. 1 and FIG. 2, only the upper sheet 20 and the lower sheet 50 are provided with the tacky layers 21, 22, 51, and 52. A step that needs attachment accuracy in order to, for example, prevent entry of moisture to the sensor body 42 is a step of attaching the lower sheet 50 (tacky layer 51) to the upper sheet 20 (tacky layer 22) to which the electrode 30 is attached. The biological sensor 100 of this embodiment can minimize the number of attachment steps that need accuracy, and suppress displacement upon production (upon assembling). This can increase production efficiency, suppress reduction in production yield that is a percentage of acceptable biological sensors 100, and reduce production cost.

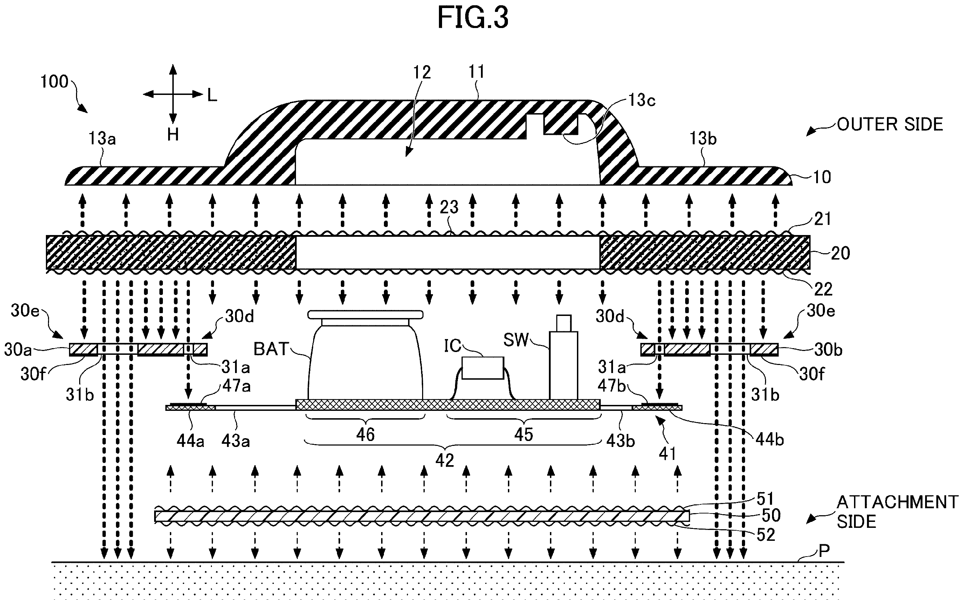

FIG. 3 is an exploded cross-sectional diagram illustrating a cross section of the biological sensor 100 of FIG. 1 in the longitudinal direction L. FIG. 3 illustrates a schematic cross section taken along line I-I' described in the sensor portion 40 of FIG. 2, and the biological sensor 100 is extended for emphasis in the height direction (thickness) thereof. The tacky layers 21 and 22 provided on the upper sheet 20 and the tacky layers 51 and 52 provided on the lower sheet 50 are denoted by wavy lines. Note that FIG. 3 illustrates a state in which the biological sensor 100 is attached to the skin of the living body P (subject) and thus the release sheet 60 of FIG. 1 is removed. As illustrated in both sides of the longitudinal direction L in FIG. 3, the outer periphery of the upper sheet 20 projects with respect to the outer periphery of the cover 10. Also, FIG. 3 illustrates an example in which a foamed sheet is used for the upper sheet 20.

As described above, the tacky force of the tacky layers 21 and 22 is higher than the tacky force of the tacky layers 51 and 52. In FIG. 3, the tacky layers 21 and 22 having a relatively high tacky force are denoted by thick wavy lines, and the tacky layers 51 and 52 having a relatively low tacky force are denoted by thin wavy lines. Also, thick dashed-line arrows vertically shown in FIG. 3 indicate that the tacky force of the tacky layers 21 and 22 is relatively high, and thin dashed-line arrows indicate that the tacky force of the tacky layers 51 and 52 is relatively low. Note that the tacky force of the tacky layer 51 may be made higher than the tacky force of the tacky layer 52, and made nearly equal to the tacky force of the tacky layer 22.

The tacky layer 52 of the lower sheet 50 is attached to the skin of the living body P, thereby fixing the biological sensor 100 to the living body P. In the biological sensor 100, a portion outward of the terminal 44 in the longitudinal direction L is attached to the skin with the tacky layer 22 having a higher tacky force, and a portion inward of the terminal 44 in the longitudinal direction L is attached to the skin with the tacky layer 52 having a lower tacky force.

The tacky layer 21 of the upper sheet 20 attaches the upper sheet 20 to the flat surface of the cover 10 on the attachment side. In the tacky layer 22 of the upper sheet 20, a portion facing the electrode 30 (30a, 30b) is attached to the electrode 30. Here, portions of the tacky layer 22 that face the through-holes 31a and 31b in the electrode 30 are exposed to the attachment side via the through-holes 31a and 31b.

The tacky layer 22 exposed from the through-hole 31b provided in the exposed portion 30e of the electrode 30 is attached to the living body P, and functions for firmly attaching the electrode 30 to the skin of the living body P. The tacky layer 22 exposed from the through-hole 31a provided in the facing portion 30d of the electrode 30 is attached to a pad 47 (47a, 47b) of the terminal 44 (44a, 44b), and functions for firmly attaching the electrode 30 to the pad 47.

For example, a surface of the electrode 30 on the attachment side includes a conductive polymer 30f, and a surface of the pad 47 is plated with gold. The conductive polymer 30f may be provided in at least the surface of the electrode 30 on the attachment side, but may be provided in both of the surfaces thereof.

Of the tacky layer 22 that does not face the flexible substrate 41 or the electrode 30, a portion facing the lower sheet 50 is attached to the tacky layer 51 of the lower sheet 50. When the tacky layers 22 and 51 are adhered to each other, the conductive polymer 30f of the electrode 30 and the pad 47a of the terminal 44 are firmly attached in a compressed state. Meanwhile, of the tacky layer 22 that does not face the flexible substrate 41 or the electrode 30, a portion not facing the lower sheet 50 is attached to the skin of the living body P.

For example, an integrated circuit IC (e.g., CPU, ASIC, or the like), a switch SW, and a battery BAT are mounted in the sensor body 42. The integrated circuit IC is configured to process biological signals obtained from the living body P and generate biological signal data. The switch SW is configured to start up the biological sensor 100. The battery BAT is configured to supply electric power to the integrated circuit IC. The integrated circuit IC and the switch SW are mounted in the parts-mounting portion 45, and the battery BAT is mounted in the battery-receiving portion 46. For example, the switch SW is a press switch. The housing space 12 is provided with a projection 13c at a position facing the switch SW. The projection 13c is for reducing the distance to a tip end of the switch SW, and for applying a pressing force from the projection 11 to the tip end of the switch SW without dispersing the pressing force.

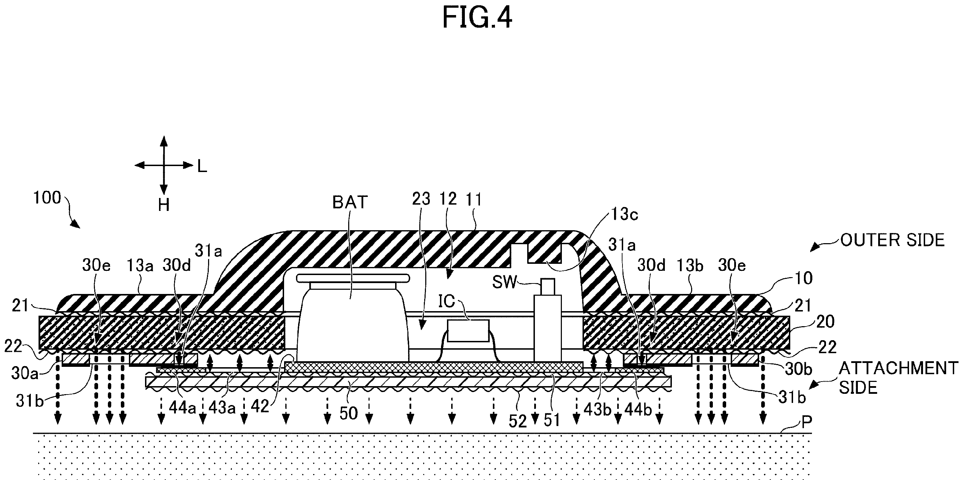

FIG. 4 is a schematic diagram illustrating a cross section of the biological sensor 100 of FIG. 1 in the longitudinal direction L. The same elements as in FIG. 3 are denoted by the same reference numerals. Similar to FIG. 3, FIG. 4 illustrates a schematic cross section taken along line I-I' described in the sensor portion 40 of FIG. 2, and the biological sensor 100 is extended for emphasis in the height direction (thickness) thereof.

In this embodiment, the outer periphery of the upper sheet 20 projects with respect to the outer periphery of the cover 10, and thus it is possible to increase an attachment area to the living body P compared to when the upper sheet 20 is formed to have the same size as the cover 10. This can increase an attachment performance (detachment endurance) of the biological sensor 100 with respect to the living body P.

When the skin is deformed by the body movement of the living body P in a state in which the biological sensor 100 is attached to the living body P, the biological sensor 100 is deformed in conformity to the deformation of the skin. At this time, because the upper sheet 20 projects with respect to the outer periphery of the cover 10, it is possible to suppress the end of the outer periphery of the cover 10 from directly touching the skin upon the deformation of the skin caused by the body movement. Therefore, it is possible to suppress skin irritation due to the outer periphery of the cover 10, and suppress the occurrence of skin pain or itching.

The skin irritation due to the outer periphery of the cover 10 is reduced as the upper sheet 20 becomes thicker. Further, because the upper sheet 20 is a foamed sheet, the pressing force applied to the skin by the outer periphery of the cover 10 can be dispersed in the upper sheet 20 even if the end of the outer periphery of the cover 10 is directed to the skin due to the deformation of the skin. As a result, the skin irritation can be reduced.

When the skin is extended by the body movement, the outer periphery of the upper sheet 20 is extended in conformity to the extension of the skin. At this time, the deformation of the tacky layer 22 of the upper sheet 20 and the foamed sheet in the upper sheet 20 reduces the stress applied to the cover 10, thereby suppressing the deformation on the tacky layer 21 side. That is, the projecting portion of the outer periphery of the upper sheet 20 can function as a buffer material that absorbs the extension of the skin, and a part of the extension of the skin can be absorbed by the upper sheet 20.

This can reduce extension of the outer periphery of the cover 10 due to the extension of the skin. Therefore, the skin can be suppressed from being pulled in a direction of contraction by a reaction force against the extension of the outer periphery of the cover 10 following the extension of the skin, and it is possible to suppress occurrence of body movement-derived pain or itching of the skin to which the outer periphery of the biological sensor 100 is attached. As a result, the attachment sensation during attachment of the biological sensor 100 to the living body P can be improved.

As described above, because the pressing force applied to the skin from the cover 10 upon the deformation of the skin by the body movement can be dispersed in the upper sheet 20, it is possible to suppress detachment of the biological sensor 100 which would otherwise occur by the action of the reaction force from the skin against the pressing force applied by the cover 10. The detachment of the biological sensor 100 from the skin is successfully suppressed, and thus it is possible to lower the tacky force of the tacky layer 22 of the upper sheet 20. This can reduce pain upon detaching the biological sensor 100 from the skin. When lowering the tacky force of the tacky layer 22 of the upper sheet 20, the tacky force of the tacky layer 21 may be made higher than the tacky force of the tacky layer 22.

In addition, the contact area of the tacky layer 22 of the upper sheet 20 with the skin can be increased compared to when the upper sheet 20 is formed to have the same size as the cover 10. This enables use of the tacky layer 22 having a lower tacky force compared to when the upper sheet 20 is formed to have the same size as the cover 10. As a result, the adhesive force per unit area of the tacky layer 22 of the upper sheet 20 can be lowered, and thus it is possible to readily detach the biological sensor 100 from the living body P without involving degradation in adhesion performance of the biological sensor 100 to the living body P. For example, it is possible to detach the biological sensor 100 from the living body P without using a tool, such as a remover or the like, and without causing pain in the living body P.

This embodiment illustrates an example in which the entire outer periphery of the upper sheet 20 projects with respect to the outer periphery of the cover 10. However, only a part of the outer periphery of the upper sheet 20 may project with respect to the outer periphery of the cover 10. In other words, the outer periphery of the upper sheet 20 at a position at which detachment of the biological sensor 100 is more likely to occur following the body movement and the like of the living body P may project with respect to the outer periphery of the cover 10.

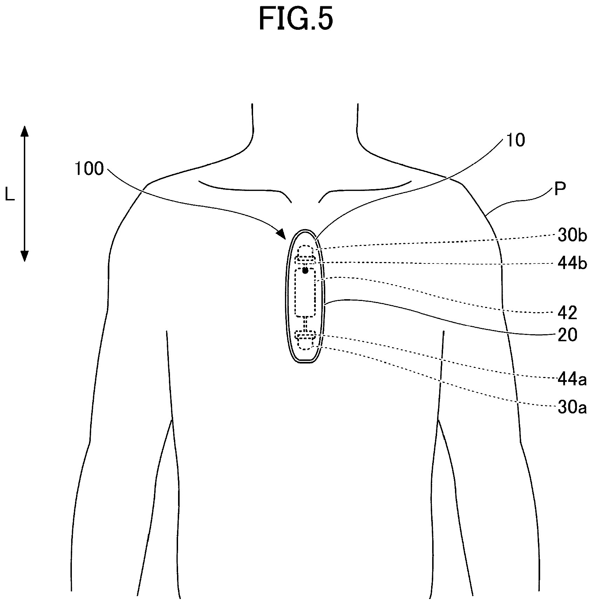

For example, as illustrated in FIG. 5, a portion of the cover 10 on the flat portion 13a side is positioned on the ventral side of the living body P at an attachment position corresponding to a NASA lead. The position on the ventral side is likely to be displaced by the body movement more greatly than in a position on the flat portion 13b side, and is more likely to cause detachment of the biological sensor 100 than in the position on the flat portion 13b side. Therefore, at least the end of the cover 10 on the flat portion 13a side in the outer periphery of the upper sheet may project with respect to the outer periphery of the cover 10.

Also, the end of the biological sensor 100 in the longitudinal direction L is likely to be detached because of being likely to be displaced by the body movement more greatly than in the end thereof in the widthwise direction W. Therefore, only both of the ends of the outer periphery of the upper sheet 20 in the longitudinal direction L may project with respect to the outer periphery of the cover 10.

As described above, by projecting the outer periphery of the upper sheet 20, with respect to the cover 10, at a position at which detachment of the biological sensor 100 is more likely to occur, the pressing force applied to the skin from the cover 10 upon the deformation of the skin by the body movement can be dispersed in the upper sheet 20. This can suppress detachment of the biological sensor 100 which would otherwise occur by the action of the reaction force from the skin against the pressing force applied by the cover 10 to the outer periphery of the upper sheet 20.

In this embodiment, the tacky layer 22 of the upper sheet 20 has moisture permeability, and thus it is possible to transfer water vapor, generated from the living body P to which the biological sensor 100 is attached, to the upper sheet 20 via the tacky layer 22. Further, the upper sheet 20 has an open-cell structure, and thus can release the water vapor entering the upper sheet 20 via the tacky layer 22, to the exterior of the biological sensor 100.

This can prevent accumulation of sweat or water vapor at the interface between the tacky layer 22 and the skin of the living body P to which the biological sensor 100 is attached. As a result, it is possible to prevent detachment of the biological sensor 100 from the skin which would otherwise occur due to the tacky force of the tacky layer 22 lowered by the action of moisture accumulated at the interface between the skin and the tacky layer 22.

Meanwhile, the lower sheet 50 is formed using a resin sheet having waterproofness. Therefore, it is possible to prevent entry of sweat or water vapor, generated from the living body P, through the lower sheet 50 toward the flexible substrate 41 in a state in which the biological sensor 100 is attached to the skin of the living body P. Also, the electrode 30 (30a, 30b) and the terminal 44 (44a, 44b) are disposed between the upper sheet 20 and the lower sheet 50 and contact each other by the tacky layers 22 and 51 in a compressed state. This can prevent entry of sweat or water vapor toward the sensor body 42 from the interface between the lower sheet 50 and the terminal 44.

As illustrated in FIG. 4, by projecting both ends of the lower sheet 50 in the longitudinal direction L with respect to the end of the terminal 44, the interface between the electrode 30 and the end of the terminal 44 may be covered by the tacky layer 51. This can prevent entry of sweat or water vapor toward the sensor body 42 from the interface between the terminal 44 and the electrode 30.

Further, the tacky layer 22 of the upper sheet 20 is exposed to the terminal 44 side from the through-hole 31a provided in the electrode 30, and thus it is possible to maintain the pressing force applied to the electrode 30 over the entire surface of the terminal 44. This can block a channel for entry of sweat or water vapor toward the sensor body 42 from the interface between the lower sheet 50 and the terminal 44 and from the interface between the terminal 44 and the electrode 30.

With the above-described structure, it is possible to prevent breakage or disconnection due to corrosion or the like that may occur in the parts mounted in the parts-mounting portion 45, such as the integrated circuit IC and the like, the battery BAT received in the battery-receiving portion 46, the interconnects, or the like. As a result, it is possible to prevent occurrence of abnormal operation of the biological sensor 100 and inability to measure biological signals.

The electrode 30 and the terminal 44 are held between the upper sheet 20 and the lower sheet 50 via the tacky layers 22 and 51. By virtue of the tacky force between the tacky layers 22 and 51, the terminal 44 and the facing portion 30d of the electrode 30 can contact each other in a compressed state, and contact resistance between the electrode 30 and the terminal 44 can be lowered.

Also, by virtue of the tacky layer 22 exposed via the through-hole 31a positioned in the facing portion 30d of the electrode 30, the facing portion 30d of the electrode 30 can contact the terminal 44 in a compressed state. Therefore, the contact resistance between the electrode 30 and the terminal 44 can be further lowered compared to when an electrode free of the through-hole 31a contacts the terminal 44.

Also, by virtue of the tacky layer 22 positioned around the exposed portion 30e, the exposed portion 30e of the electrode 30 can contact the skin of the living body P in a compressed state. Further, by virtue of the tacky layer 22 exposed via the through-hole 31b positioned in the exposed portion 30e of the electrode 30, the exposed portion 30e of the electrode 30 can contact the skin of the living body P in a compressed state. Therefore, it is possible to increase the pressing force applied to the skin not only in the periphery of the exposed portion 30e but also in a center portion of the exposed portion 30e.

As described above, by holding the electrode 30 and the terminal 44 between the upper sheet 20 and the lower sheet 50 via the tacky layers 22 and 51, it is possible to lower the contact resistance between the electrode 30 and the terminal 44. Also, by virtue of the tacky layer 22 around the electrode 30 and the tacky layer 22 exposed from the through-hole 31b, the electrode 30 can contact the skin in a compressed state, and the contact resistance between the electrode 30 and the skin can be lowered. As a result, it is possible to increase detection accuracy of biological signals by the biological sensor 100.

When the contact resistance between the electrode 30 and the terminal 44 and the contact resistance between the electrode and the skin of the living body P can be lowered to be equal to or lower than respective predetermined values, the through-holes 31a and 31b may not be provided in the electrode 30. Alternatively, based on evaluation of values of the contact resistance, either the through-hole 31a in the facing portion 30d of the electrode 30 or the through-hole 31b in the exposed portion 30e may be provided.

The tacky layer 51 of the lower sheet 50 is attached to the upper sheet 20 so as to cover the narrow portion 43 disposed flatly along the lower sheet 50, and holds and fixes the narrow portion 43 between the upper sheet 20 and the lower sheet 50. The upper sheet 20 has the through-hole 23 through which the sensor body 42 can be inserted. Thus, by disposing the sensor body 42 on the attachment side of the upper sheet 20, it is possible to hold the sensor body 42 between the upper sheet 20 and the lower sheet 50 without bending the narrow portion 43 in the height direction H.

Further, the tacky layer 51 of the lower sheet 50 is attached to the flat surface of the sensor body 42 on the attachment side, and fixes the sensor body 42 in a state of being housed in the housing space 12 provided in the cover 10. This enables the sensor body 42 and the narrow portion 43 to vibrate together even if the biological sensor 100 is vibrated by the body movement of the living body P to which the biological sensor 100 is attached. Thus, it is possible to prevent concentration of stress in the narrow portion 43. As a result, it is possible to prevent disconnection due to deformation of the interconnects of the narrow portion 43.

For example, by increasing the tacky force of the tacky layer 22 positioned at both ends in the longitudinal direction L, even if stress is applied to the attachment surface of the biological sensor 100 to the skin by the body movement of the living body P, it is possible to prevent detachment of the tacky layer 22 from the skin. This can prevent increasing in the contact resistance between the electrode 30 and the skin, and prevent reduction in measurement accuracy for biological signals.

Meanwhile, the tacky force of the tacky layer 52 is low, and thus it is possible to reduce pain upon detaching the biological sensor 100 from the living body P. As a result, it is possible to prevent reduction in measurement accuracy upon measurement of biological signals or inability to perform measurement while reducing pain upon detaching the biological sensor 100 from the living body P.

The upper sheet 20 is formed of a foamed sheet, and thus the upper sheet 20 can absorb a part of stress applied to the attachment surface of the biological sensor 100 to the skin, the stress being generated by the action of the deformation of the skin caused by the body movement of the living body P. By reducing the stress applied to the attachment surface to the skin, it is possible to reduce tightness felt by the living body P upon the deformation of the skin, and improve attachment sensation during attachment of the biological sensor 100.

Improvement in the attachment sensation during attachment of the biological sensor 100 can be achieved by forming the upper sheet 20 with a foamed material having flexibility. Such improvement can also be achieved by decreasing the thickness of the peripheral edge of the cover 10 (especially the thicknesses at both sides in the longitudinal direction L). By forming the upper sheet 20 with a foamed material and decreasing the thickness of the peripheral edge of the cover 10, it is possible to increase effects of improving attachment sensation during attachment of the biological sensor 100.

In FIG. 2 to FIG. 4, the flat portions 13a and 13b of the cover 10 have a shape that covers the electrodes 30a and 30b in a plan view. However, the flat portions 13a and 13b may have a shape that does not cover a part or the entirety of the electrodes 30a and 30b in a plan view. As the areas of the flat portions 13a and 13b decrease, it is possible to increase the area of a portion projecting from the cover 10 in the outer periphery of the upper sheet 20, and improve efficiency of releasing vapor generated by sweat or the like. As a result, detachment of the biological sensor 100 from the living body P can be successfully suppressed.

Further, by reducing the lengths of the flat portions 13a and 13b, it is possible to reduce the length of the upper sheet 20 in the longitudinal direction to an extent that the electrodes 30a and 30b are not exposed. As a result, it is possible to reduce the size of the biological sensor 100.

FIG. 5 is an explanatory diagram illustrating a state in which the biological sensor 100 of FIG. 1 is attached to the chest of the living body P. For example, the biological sensor 100 is attached to the living body P at a position of what is called a NASA lead. Specifically, the biological sensor 100 is attached such that the longitudinal direction L thereof is aligned to the sternum of the living body P, with the electrode 30b disposed at an upper side and the electrode 30a disposed at a lower side. In a state in which the biological sensor 100 is attached to the living body P, the outer periphery of the upper sheet 20 projects with respect to the outer periphery of the cover 10. The biological sensor 100 may be attached at a position of a CM5 lead or a position of a CC5 lead.

The electrodes 30a and 30b of the biological sensor 100 are in contact with the body surface of the living body P in a compressed state by attachment to the living body P via the tacky layers 22 and 52 of FIG. 4. In this state, the biological sensor 100 obtains biological signals from the living body P, such as electrocardiographic signals and the like. For example, the biological sensor 100 stores the obtained biological signal data in a non-volatile memory mounted in the parts-mounting portion 45, such as a flash memory or the like.

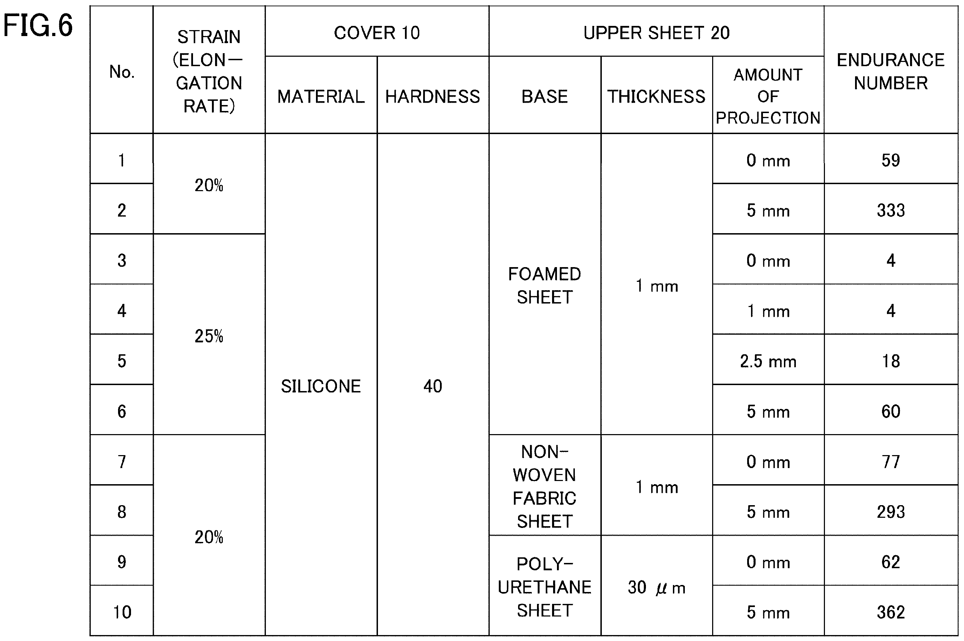

FIG. 6 is a table describing examples of evaluation results of detachment endurance of the biological sensor 100 against the stretch of the skin. Evaluation on endurance was performed in a state in which the upper sheet 20 and the cover 10 were sequentially attached to a bio skin plate having similar stretchability of the skin. The cover 10 used for the evaluation was silicone having a hardness of 40. The upper sheet 20 used for the evaluation was a foamed sheet having a thickness of 1 mm (e.g., FOLEC available from INOAC CORPORATION), a non-woven fabric sheet having a thickness of 1 mm (e.g., base fabric EW for medical patch available from JAPAN VILENE COMPANY, LTD.), or a polyurethane sheet having a thickness of 30 um (e.g., ESMER URS available from Nihon Matai Co., Ltd.). The amount of projection of the upper sheet 20 with respect to the cover 10 was 0 mm, 1 mm, 2.5 mm, or 5 mm.

In the evaluation, an end portion corresponding to one longitudinal end of the cover 10 (on the flat portion 13b side) was fixed to the bio skin plate. Then, the entirety of the bio skin plate was repeatedly stretched. Also, in the evaluation, strain (elongation rate) of the bio skin plate was set to 20% or 25%, and was stretched at a frequency of 20 times per minute. Then, the number of times of stretching until the other longitudinal end of the upper sheet 20 (corresponding to the flat portion 13a side) was released from the bio skin plate, was counted, and the counted number was defined as an endurance number.

For the foamed sheet, the endurance number with the strain of the bio skin plate being 20% was 59 when the amount of projection was 0 mm and 333 when the amount of projection was 5 mm. For the foamed sheet, the endurance number with the strain of the bio skin plate being 25% was 4 when the amount of projection was 0 mm and 1 mm, 18 when the amount of projection was 2.5 mm, and 60 when the amount of projection was 5 mm. For the non-woven fabric sheet, the endurance number with the strain of the bio skin plate being 20% was 77 when the amount of projection was 0 mm and 293 when the amount of projection was 5 mm. For the polyurethane sheet, the endurance number with the strain of the bio skin plate being 20% was 62 when the amount of projection was 0 mm and 362 when the amount of projection was 5 mm.

In view of correlation between: the strain of the bio skin plate and the endurance number; and the time taken for the biological sensor 100 attached to the living body P to be detached from the living body P by the action of the body movement, it was found that when the amount of projection was 5 mm or more, the biological sensor 100 was able to continue to measure biological information for 72 hours or longer without being detached.

In the embodiments as illustrated in FIG. 1 to FIG. 5, detachment of the biological sensor 100 from the living body P can be successfully suppressed by projecting at least a part of the outer periphery of the upper sheet 20 with respect to the outer periphery of the cover 10. As a result, it is possible to extend the time for which the biological sensor 100 measures biological information, compared to when the upper sheet 20 is formed to have the same size as the cover 10.

Because the upper sheet 20 projects with respect to the outer periphery of the cover 10, it is possible to suppress the edge of the outer periphery of the cover 10 from directly touching the skin upon the deformation of the skin caused by the body movement. Therefore, it is possible to suppress skin irritation (occurrence of pain) due to the outer periphery of the cover 10.

Because the thickness of the upper sheet 20 is larger than the thickness of the outer periphery of the cover 10, it is possible to reduce the skin irritation caused by the outer periphery of the cover 10. Further, because the upper sheet 20 is a foamed sheet, the pressing force applied to the skin by the outer periphery of the cover 10 can be dispersed in the upper sheet 20 even if the end of the outer periphery of the cover 10 is directed to the skin due to the deformation of the skin. As a result, the skin irritation can be reduced.

Because the projecting portion of the outer periphery of the upper sheet 20 functions as a buffer material that absorbs the extension of the skin, the skin can be suppressed from being pulled in a direction of contraction by the reaction force against the extension of the outer periphery of the cover 10 following the extension of the skin. Therefore, it is possible to suppress occurrence of body movement-derived pain of the skin to which the outer periphery of the biological sensor 100 is attached. As a result, the attachment sensation during attachment of the biological sensor 100 can be improved.

Because the pressing force applied to the skin by the cover 10 upon the deformation of the skin caused by the body movement can be dispersed in the upper sheet 20, it is possible to suppress detachment of the biological sensor 100 which would otherwise occur by the action of the reaction force from the skin against the pressing force applied by the cover 10. Because detachment of the biological sensor 100 from the skin is successfully suppressed, it is possible to reduce the tacky force of the tacky layer 22 of the upper sheet 20. As a result, it is possible to reduce skin irritation due to the tacky layer 22.

Because the contact area of the tacky layer 22 of the upper sheet 20 with the skin can be increased, it is possible to reduce the tacky force of the tacky layer 22. Thus, the biological sensor 100 can be readily detached from the living body P without causing pain in the living body P.

The biological sensor 100 includes the upper sheet 20 and the lower sheet 50 having different tacky forces and moisture permeability (waterproofness). Therefore, the moisture permeability and the tacky force applied to the living body P can be made different, for example, between the portion where the electrode 30 is formed and the portion where the parts are mounted. It is possible to employ different tacky forces and moisture permeability (waterproofness) in accordance with positions in the biological sensor. As a result, it is possible to provide the biological sensor 100 that satisfactorily achieves both release of moisture generated from the living body P and prevention of entry of moisture to the sensor body 42.

As a result, it is possible to prevent detachment of the biological sensor 100 from the living body P and prevent breakage of the biological sensor 100 due to entry of moisture to the sensor body 42. That is, it is possible to prevent inability to measure biological signals due to sweat or water vapor generated from the living body in which the biological sensor is mounted.

Because it is possible to minimize the number of steps that need attachment accuracy in order to prevent entry of moisture to the sensor body 42, it is possible to increase production efficiency in an assembling step of the biological sensor 100. This can suppress reduction in production yield that is a percentage of acceptable biological sensors 100, and reduce production cost.

By providing the upper sheet 20 with the through-hole 23 through which the sensor body 42 can be inserted and disposing the sensor body 42 on the attachment side of the upper sheet 20, it is possible to hold the sensor body 42 between the upper sheet 20 and the lower sheet 50 without bending the narrow portion 43 in the height direction H. This can prevent application of mechanical stress in a state in which the narrow portion 43 is bent, and prevent disconnection of the narrow portion 43.

By holding the electrode 30 and the terminal 44 between the tacky layers 22 and 51 in a compressed state, it is possible to prevent entry of sweat or water vapor to the sensor body 42 from the interface between the electrode 30 and the terminal 44. As a result, it is possible to prevent breakage of the parts mounted in the sensor body 42, and prevent occurrence of abnormal operation of the biological sensor 100.

Because the upper sheet 20 is a foamed sheet having flexibility, the upper sheet 20 can absorb a part of stress applied to the attachment surface of the biological sensor 100 to the skin, the stress being generated by the body movement of the living body P (stretching, bending, or twisting). Therefore, it is possible to improve attachment sensation during attachment of the biological sensor 100. Also, by virtue of the upper sheet 20 that is a foamed sheet having an open-cell structure, it is possible to efficiently release moisture generated from the living body P. This can prevent accumulation of moisture at the interface between the tacky layer 22 and the skin, and prevent detachment of the biological sensor 100 from the skin. As a result, it is possible to prevent inability to measure biological signals from the living body P by the biological sensor 100 mounted in the living body P.

By increasing the tacky force of the tacky layer 22 positioned outward in the longitudinal direction L, even if stress is applied to the attachment surface of the biological sensor 100 to the skin by the body movement of the living body P, it is possible to prevent detachment of the tacky layer 22 from the skin. This can prevent increasing in the contact resistance between the electrode 30 and the skin, and prevent reduction in measurement accuracy for biological signals.

Meanwhile, the tacky force of the tacky layer 52 is low, and thus it is possible to reduce pain upon detaching the biological sensor 100 from the living body P. As a result, it is possible to prevent reduction in measurement accuracy upon measurement of biological signals or inability to perform measurement while reducing pain upon detaching the biological sensor 100 from the living body P.

By decreasing the thicknesses of the flat portions 13a and 13b compared to the thickness of the projection 11, the flat portions 13a and 13b can deform in conformity to the deformation of the body surface caused by the body movement of the living body P to which the biological sensor 100 is attached. This can reduce the stress applied to the flat portions 13a and 13b by the body movement, and prevent detachment of the biological sensor 100 from the skin.

By exposing the tacky layer 22 from the through-hole 31b of the electrode 30 to the attachment side, the exposed portion 30e of the electrode 30 can contact the skin of the living body P in a compressed state. Therefore, it is possible to increase the pressing force applied to the skin not only in the periphery of the exposed portion 30e but also in the vicinity of the electrode 30 that is a center portion of the exposed portion 30e. As a result, it is possible to reduce the contact resistance between the electrode 30 and the skin, and further increase detection accuracy of biological signals by the biological sensor 100.

Also, by exposing the tacky layer 22 from the through-hole 31a of the electrode 30 to the attachment side, the facing portion 30d of the electrode 30 can contact the terminal 44 in a compressed state by virtue of the tacky layer 22 exposed from the through-hole 31a. As a result, the contact resistance between the electrode 30 and the terminal 44 can be further lowered compared to when an electrode free of the through-hole 31a contacts the terminal 44. This can further increase detection accuracy of biological signals by the biological sensor 100.

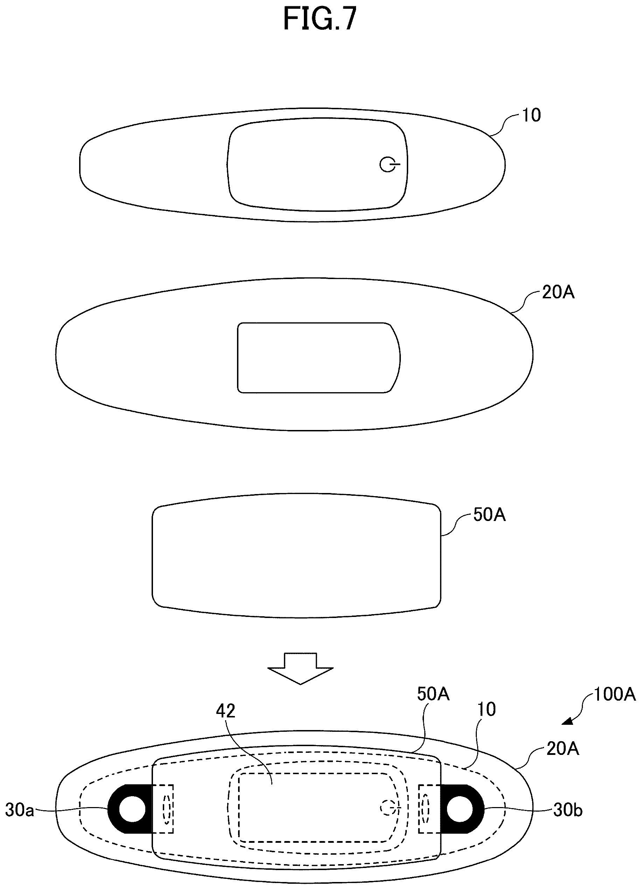

FIG. 7 is a plan diagram illustrating examples of shapes of a cover, an upper sheet, and a lower sheet of a biological sensor according to a second embodiment. The same elements as in the first embodiment are denoted by the same reference numerals, and detailed description thereof will be omitted. A biological sensor 100A of this embodiment includes an upper sheet 20A and a lower sheet 50A instead of the upper sheet 20 and the lower sheet 50 of the biological sensor 100 of FIG. 1. The other configurations and functions of the biological sensor 100A are similar to the configurations and functions of the biological sensor 100 as illustrated in FIG. 1 to FIG. 5.

For example, the upper sheet 20A is formed using the polyurethane sheet as referred to in FIG. 6. In order to further increase detachment endurance of the biological sensor 100A, the outer periphery of the upper sheet 20A projects from the outer periphery of the cover 10 by about 10 mm. Also, the outer periphery of the lower sheet 50A in the lateral direction is formed to be positioned between the outer periphery of the cover 10 and the outer periphery of the upper sheet 20A. In this manner, the outer periphery of the lower sheet 50A in the lateral direction is adhered to the upper sheet 20A.

Similar to the lower sheet 50 of the first embodiment, the lower sheet 50A is formed using, for example, a resin sheet having a thickness of about several tens of micrometers through about 100 um. For example, the lower sheet 50A may be formed to have approximately the same thickness as the thickness of the upper sheet 20A. Alternatively, the lower sheet 50A may be formed to have a thickness that gives approximately the same elastic modulus as in the upper sheet 20A.

By adhering the outer periphery of the lower sheet 50A in the lateral direction to the upper sheet 20A, the outer periphery of the lower sheet 50A deforms in conformity to the outer periphery of the upper sheet 20A even if the biological sensor 100A attached to the living body is repeatedly bent by the body movement of the living body. This can prevent formation of wrinkles in the outer periphery of the lower sheet 50A, and prevent the wrinkles from forming a gap between the lower sheet 50A and the upper sheet 20A. As a result, it is possible to prevent entry of moisture to the sensor body 42 from between the lower sheet 50A and the upper sheet 20A.

Meanwhile, when the outer periphery of the lower sheet 50A in the lateral direction is inward of the outer periphery of the cover 10, the outer periphery of the lower sheet 50A in the lateral direction is adhered to the outer periphery of the cover 10. Bending rigidity of the cover 10 is higher than bending rigidity of the lower sheet 50A. Therefore, when the biological sensor 100A attached to the living body is repeatedly bent by the body movement of the living body, wrinkles may form in the lower sheet 50A due to the difference in bending stress between the cover 10 and the lower sheet 50A.

Similar to the first embodiment, this embodiment can provide a biological sensor that can be suppressed from being detached from the living body and can suppress reduction in attachment sensation due to the deformation of the skin caused by the body movement of the living body.

Moreover, in this embodiment, the outer periphery of the lower sheet 50A in the lateral direction is positioned between the outer periphery of the cover 10 and the outer periphery of the upper sheet 20A. This can prevent formation of wrinkles in the outer periphery of the lower sheet 50A even if the biological sensor 100A is repeatedly bent by the body movement of the living body, and also prevent formation of a gap between the lower sheet 50A and the upper sheet 20A. As a result, it is possible to prevent entry of moisture to the sensor body 42 from between the lower sheet 50A and the upper sheet 20A, and prevent breakage of the biological sensor 100A due to entry of moisture.

Although the present invention has been described above based on the embodiments, the present invention should not be construed as being limited to the specifically disclosed embodiments. The above-described matters can be changed within a scope in which the gist of the present invention is not impaired.

Aspects of the present invention are, for example, as follows.
(1) A biological sensor, including:
   a sensor body configured to obtain biological information;
   a cover member having a housing space and an opening of the housing space, the sensor body being housed in the housing space;
   a first sheet member provided to face the opening in the cover member; and
   a second sheet member provided to face the cover member with the first sheet member being between the second sheet and the cover member, in which
   at least a part of an outer periphery of the first sheet member projects with respect to outer peripheries of the cover member and the second sheet member.
(2) The biological sensor according to (1), in which
   the cover member and the first sheet member have a thin and long shape, and
   either or both of longitudinal ends of the outer periphery of the first sheet member project with respect to the outer periphery of the cover member.
(3) The biological sensor according to (1) or (2), in which
   stretchability of the first sheet member is higher than stretchability of the cover member.
(4) The biological sensor according to claim 3, in which
   the first sheet member is formed using a foamed sheet, a polyurethane sheet, or a non-woven fabric sheet.
(5) The biological sensor according to any one of claims 1 to 4, in which
   moisture permeability of the first sheet member is higher than moisture permeability of the cover member.
(6) The biological sensor according to any one of claims 1 to 5, in which
   moisture permeability of the first sheet member is higher than moisture permeability of the second sheet member.
(7) The biological sensor according to any one of claims 1 to 6, in which
   the outer periphery of the first sheet member projects with respect to the outer periphery of the second sheet member.
(8) The biological sensor according to any one of claims 1 to 7, in which
   the outer periphery, in a lateral direction, of the first sheet member having the thin and long shape projects the outer periphery of the cover member in the lateral direction, and
   the outer periphery of the second sheet member in the lateral direction is positioned between the outer periphery of the cover member and the outer periphery of the first sheet member.

The present application claims priority to Japanese Patent Application No. 2021-157511, filed on September 28, 2021 with the Japan Patent Office and Japanese Patent Application No. 2022-51477, filed on March 28, 2022 with the Japan Patent Office, and the contents of the above applications are incorporated herein by reference in their entirety.

### REFERENCE SIGNS LIST

10 cover
11 projection
12 housing space
13 (13a, 13b) flat portion
13c projection
20, 20A upper sheet
21, 22 tacky layer
23 through-hole
30 (30a, 30b) electrode
30d facing portion
30e exposed portion
30f conductive polymer
31a, 31b through-hole
40 sensor portion
41 flexible substrate
42 sensor body
43 (43a, 43b) narrow portion
44 (44a, 44b) terminal
45 parts-mounting portion
46 battery-receiving portion
47 (47a, 47b) pad
50, 50A lower sheet
51, 52 tacky layer
60 release sheet
100, 100A biological sensor
BAT battery
IC integrated circuit
P living body
SW switch

## Claims

1. A biological sensor, comprising:
a sensor body configured to obtain biological information;
a cover member having a housing space and an opening of the housing space, the sensor body being housed in the housing space;
a first sheet member provided to face the opening in the cover member; and
a second sheet member provided to face the cover member with the first sheet member being between the second sheet and the cover member, wherein
at least a part of an outer periphery of the first sheet member projects with respect to outer peripheries of the cover member and the second sheet member.

2. The biological sensor according to claim 1, wherein
the cover member and the first sheet member have a thin and long shape, and
either or both of longitudinal ends of the outer periphery of the first sheet member project with respect to the outer periphery of the cover member.

3. The biological sensor according to claim 1 or 2, wherein
stretchability of the first sheet member is higher than stretchability of the cover member.

4. The biological sensor according to claim 3, wherein
the first sheet member is formed using a foamed sheet, a polyurethane sheet, or a non-woven fabric sheet.

5. The biological sensor according to claim 1 or 2, wherein
moisture permeability of the first sheet member is higher than moisture permeability of the cover member.

6. The biological sensor according to claim 1 or 2, wherein
moisture permeability of the first sheet member is higher than moisture permeability of the second sheet member.

7. The biological sensor according to claim 1 or 2, wherein
the outer periphery of the first sheet member projects with respect to the outer periphery of the second sheet member.

8. The biological sensor according to claim 1 or 2, wherein
the outer periphery, in a lateral direction, of the first sheet member having the thin and long shape projects with respect to the outer periphery of the cover member in the lateral direction, and
the outer periphery of the second sheet member in the lateral direction is positioned between the outer periphery of the cover member and the outer periphery of the first sheet member.
